(19)

**Europäisches Patentamt**
**European Patent Office**
**Office européen des brevets**

(11)  **EP 2 932 978 A1**

(12)  **EUROPEAN PATENT APPLICATION**
published in accordance with Art. 153(4) EPC

(43) Date of publication:
**21.10.2015 Bulletin 2015/43**

(21) Application number: **13862358.2**

(22) Date of filing: **12.12.2013**

(51) Int Cl.:
**A61K 47/04** (2006.01)     **A61K 9/00** (2006.01)
**C01B 33/14** (2006.01)     **A61P 35/00** (2006.01)

(86) International application number:
**PCT/CN2013/089312**

(87) International publication number:
**WO 2014/090185 (19.06.2014 Gazette 2014/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.12.2012  CN 201210537252**

(71) Applicant: **Graduate School at Shenzhen, Tsinghua University**
**Guangdong 518055 (CN)**

(72) Inventors:
 • **ZHANG, Xuxu**
  **Shenzhen**
  **Guangdong 518055 (CN)**
 • **KANG, Feiyu**
  **Shenzhen**
  **Guangdong 518055 (CN)**

(74) Representative: **Decamps, Alain René François et al**
**Office Kirkpatrick S.A.**
**Avenue Wolfers, 32**
**1310 La Hulpe (BE)**

(54) **APPLICATION OF SILICON DIOXIDE AEROGEL IN PHARMACY**

(57)  The invention relates to an application of silicon dioxide aerogel as a nano-drug carrying system in pharmacy. The silicon dioxide aerogel has a nanosized drug carrying hole structure, and is a nanosized pharmaceutical excipient capable of realizing a physical drug carrying scale below 100nm.

S4800 3.0kV 7.1mm x30.0k SE(U,LA0) 5/31/2011          1.00μm

Fig.3

EP 2 932 978 A1

**Description**

Field of the Invention

The invention relates to the application of silicon dioxide aerogel and specifically relates to the application of silicon dioxide aerogel as a nano-drug carrying system in pharmacy.

Background of the Invention

With the extensive application of combinatorial chemistry and a high-throughput screening technology in research and development of new drugs, the development speed of new compound entities is becoming faster and faster. However, according to statistics, in many new compound entities with physiological activity, hydrophobic compounds become more and more, the proportion is as high as about 40%, and the proportion of chemically synthesized candidate compounds is as high as 60%. It is worth mentioning that, in the development stage, up to 40%-70% of the compounds cannot produce a sufficient curative effect due to insufficient solubility. Thus, the research and development of brand new drugs has the characteristics of long period, high input and high risk. If many candidate compounds are difficult to be orally absorbed or less liable to being directly prepared into injections due to the problem of solubility, the stop or (and) termination of the development is caused, thereby inevitably leading to huge economic loss and waste of resources. Statistical data show that the lag of pharmaceutical preparation technologies seriously impedes the development of new drugs. Some major drug species with a very high market share, such as paclitaxel (with annual sales of more than 10 billion dollars), insulin and the like, only have the dosage form of injections and have relatively high toxicity and side effects, while the oral preparations and other dosage forms which have better anticipated effects and are very expected in the market have not been developed successfully till now. Taking the solubility of the drug as an example, due to the defect of poor water solubility, about 40% of the candidate drugs cannot be marketed, and there has not been any breakthrough progress in solving the problem of solubility. It is estimated that about 65 billion dollars of the drugs lead to a serious imbalance between the proportion of the treatment cost and the curative effect due to poor bioavailability every year around the world. However, many hardly soluble drugs have a very strong bioactivity actually and thus have a good curative effect in the treatment of tumors, cardiovascular diseases and the like. Thus, how to improve the solubility and absorptivity of the drugs also becomes the hotspot and the difficult point in the research of pharmacy, and it is urgent to develop a new preparation technology and the dosage form to solve the problem.

The appearance of a nano-technology provides broad prospects for the development of biological medicines. In recent decades, various nano-drug delivery systems, such as nanoemulsions, lipid nanoparticles, nanomicelles, nanogels, nanocrystals, albumin nanoparticles and the like, emerged one after another. A nanoparticle drug delivery system provides a new carrier for delivering the hardly soluble drug, so that the water solubility of the drug can be increased, the dispersivity is improved, the advantages of improving oral absorption and bioavailability of the drug and strengthening targeting property and the like of the drug are realized, and the curative effect of the drug is further improved; meanwhile, the system can reduce toxicity and side effects caused by high distribution of a solubilizing agent, a cosolvent and other excipients and a non-target area; and furthermore, the system can also enable the water-soluble drug, such as an injection drug delivery preparation of macromolecular polypeptide and protein, to be long-acting, improve the stability and realize the advantages of small irritation, low toxicity and side effects and the like.

In the international pharmaceutical field, anti-tumor drugs have been invented for decades and "novel drugs for drug-carrying system" have been started for decades. Due to the continuous use of traditional pharmaceutical excipients, the purposes of low toxicity and high efficiency are not achieved. Thus, only few injection type modified drugs, such as liposome type drugs for injection, albumin paclitaxel and the like, appeared in the past many years, while oral anti-tumor first-line drugs, in particular cytotoxic drugs, are still absent. At present, although research reports about the nanoparticles are not few, the problem of low oral bioavailability of the anti-tumor drugs cannot be fundamentally solved. After oral administration, a large part of nanoparticles cannot be absorbed and are discharged out of a human body, and only a small part of the nanoparticles are absorbed. If the drug absorption fluctuates at a low level, the error in percentage of the absorption dose will be significant. As for a given dose, if the intake of microparticles exceeds a predicted value, the toxicity will be produced; and if the absorption amount is relatively small or the concentration of the drug is lower than the treatment dose range, treatment failure will be caused.

In the aspect of technical indexes, the studies made by people in many years have proved that the structure at a nanosize of 10-100nm is most conductive to absorption of the drug in the human body (human capillaries are at the micron-size), but in the aspect of pharmaceutical excipients, all the current legal pharmaceutical excipients do not achieve the standard (see *Handbook of Pharmaceutical Excipients*). In the current hot studies of the "nano-drugs", some researchers use various methods to prepare the nano-drugs and nano-liposomes and the like, but in some drugs, the physical scale can only achieve the micron-size (the scale above 100nm cannot be considered as the nano-size in material science), and the drug carrying rate and the encapsulation rate required for realizing the medicinal effect cannot be met to a greater extent.

Aerogel generally refers to a nano-porous network structure constituted by mutual aggregation of nanosized ultramicroparticles, and a lightweight nano-solid material of a gas-state dispersion medium is filled in network holes. The aerogel is a solid, but 90% of the aerogel is constituted by gas, and the appearance seems like cloud. Sometimes, the aerogel is also called as "solid smoke" or "blue smoke" due to translucent color and ultralight weight.

The most common aerogel is silicon dioxide aerogel, and the silicon dioxide aerogel is a lightweight nano-porous amorphous solid material with very excellent heat protection and heat insulation performances and draws universal attention of people in the fields of heat insulation, heat preservation, biosensors, catalytic and optical materials and the like. But the precedent of applying the silicon dioxide aerogel in the field of pharmacy as the nano-drug carrying system has not been found up to now.

Summary of the Invention

Through studies, the inventor found that silicon dioxide aerogel has not only a particle size scale of less than 100nm, which is strictly defined in material science, but also an independent spatial structure of less than 100nm, each millimeter of thickness achieves 10000 layers of nanoholes, the spatial (air) volume for carrying a drug is above 90%, and the highest drug carrying rate in the history can be achieved. The silicon dioxide aerogel owns huge specific surface area and stable nano-aperture which are necessary for improving the bioavailability of drugs, and can be used for performing nano-dispersion on various types of drugs by "self-assembly", "cross-linking" and other ways so as to form independent "nano-dispersions" which cannot be aggregated with the carried drugs, realize the "nano-drugs" and the "nanocrystallization " of the various drugs with universality in a real sense and directly solve the international difficult problems that the drugs cannot be formed due to aggregation in the studies of micronano-drugs, hardly soluble drugs are very difficult to improve the bioavailability and the like in the science of preparations.

Through further studies, the inventor further discovered that by applying the silicon dioxide aerogel to the field of pharmacy, the effects of greatly improving the bioavailability of the drugs and reducing the toxicity and the side effects are realized for a variety of dosage forms, and the excellent performances are also shown in solid dispersion, sustained release, controlled release and the like. The silicon dioxide aerogel surpasses all the existing pharmaceutical excipients at home and abroad due to huge specific surface area, unprecedented drug carrying rate, high biological safety and excellent biocompatibility and biological inertia, and can be applied to chemical drugs, protein and polypeptide drugs and natural drugs, and can be applied to water-soluble, alcohol-soluble, lipid-soluble and other dosage forms.

The invention aims at providing a new application of silicon dioxide aerogel.

The new application of the silicon dioxide aerogel provided by the invention refers to the application of the silicon dioxide aerogel as a nano-drug carrying system in pharmacy, wherein the nano-drug carrying system refers to a nanoparticle drug carrying system with the diameter of less than 100nm, which is formed in the form of adsorbing a carried drug in the holes of the silicon dioxide aerogel, and the silicon dioxide aerogel has the porosity of 95-99%, the aperture of 10-50nm, the specific surface area of 200-1000$m^2$/g, the density of 3-300kg/$m^3$ and the diameter of colloidal particles constituting a network of 1-50nm.

Further, the application refers to the application of the silicon dioxide aerogel as the nano-drug carrying system in the preparation of oral preparations.

Even further, the application refers to the application of the silicon dioxide aerogel as the nano-drug carrying system in the preparation of oral anti-tumor drugs.

Further, the mass ratio of the carried drug to the silicon dioxide aerogel is 1:0.5-20.

Further, the silicon dioxide aerogel is hydrophilic silicon dioxide aerogel or the silicon dioxide aerogel has hydrophilic properties, which are obtained after heat treatment of hydrophobic silicon dioxide aerogel.

Even further, the temperature for heat treatment is 300-1000°C.

Further, the application of the silicon dioxide aerogel as the nano-drug carrying system in pharmacy is implemented through the following way:

When the carried drug is a soluble drug, the carried drug is firstly prepared into a saturated or unsaturated solution, and then the silicon dioxide aerogel is added for adsorption; and when the carried drug is a hardly soluble or insoluble drug, the carried drug is firstly prepared into a suspension, and then the silicon dioxide aerogel is added for adsorption or the carried drug and the silicon dioxide aerogel are prepared into the suspension together.

In addition, sometimes, some more complex dosage forms still need to be used to prevent degradation of the drug and achieve the requirement of controlled release or targeted treatment. Thus, the silicon dioxide aerogel can be applied to the existing preparation technologies, such as micro-pelletization, granulation, spray drying or freeze drying, thereby preparing the nano-suspension into a solid preparation.

The invention has the following benefits:

compared with the prior art, the invention has the following advantages:

1. The invention found a new pharmaceutical excipient in the field of pharmacy, the pharmaceutical excipient is not currently popular nanoparticle material or nanopowder, but is a drug carrying hole new structure which really realizes nanosize. The physical drug carrying scale below 100nm which cannot be realized by any one excipient in the current pharmaceutical excipients is realized, and the blank in nanosized pharmaceutical excipients at home and abroad is filled up, thereby having universality significance in the creation of the dosage forms of a variety of drugs, being capable of promoting relatively fast production of a series of unprecedented new dosage forms of the drugs, greatly shortening the development process of the developing drugs, forming a new drug research and development mode, fundamentally breaking through the existing modes and the methods for creating the drugs and realizing large-scale creation of the new drugs. The physical scale of polypeptide gene and protein type drugs is below 10nm, and the application of such pharmaceutical excipients brings new hope to such hot studies.

2. As for nanoparticles prepared by taking the silicon dioxide aerogel of the invention as a carrier material, the drug carrying amount can be above 90%, which is unmatched by the existing liposome nanoparticles, polymer nanoparticles and the like, and the drug carrying amount can be comparable to that of the nanocrystal type drug suspension. But the manufacturing method is simpler and the cost is lower.

3. In the nanoparticles prepared by taking the silicon dioxide aerogel of the invention as the carrier material, the active drug is loaded in numerous nanosized holes of the silicon dioxide aerogel to form independent "nano-dispersions" which cannot be aggregated, and the structure is very stable, so that the international difficult problems that the drugs cannot be formed due to aggregation in the studies of micronano-drugs, hardly soluble drugs are very difficult to improve the bioavailability and the like in the science of preparations are directly solved.

4. The nanoparticles with the diameter of below 100nm can be prepared by taking the silicon dioxide aerogel of the invention as the carrier material, the diameter range has achieved the nanoscale in the scope of material science, while the diameter of the existing nanoparticles cannot achieve this scale. Although the particles with the diameter of less than $1\mu m$ are called as nanoparticles, people tend to develop the particles with the particle size of less than 100nm. Because these particles can represent some unique physical properties, the potential different and usable biological properties are shown. For example, by being limited by microcirculation of capillary vessels of an organism and cell barriers, the optimal particle size of pharmaceutical particles which can enter blood circulation and be absorbed by the organism is 10-100nm. Thus, the nanoparticles prepared by taking the silicon dioxide aerogel of the invention as the carrier material achieve a qualitative leap in the aspect of bioavailability, thereby creating conditions for the preparation of oral preparations from the hardly soluble drugs.

5. The nanosized drugs prepared by taking the silicon dioxide aerogel of the invention as the carrier material realize a brand new oral administration mechanism which takes nano-intake as a main absorption way, greatly increase the solubility of the hardly soluble drugs through the brand new structure of "nano-solid dispersions", break through the international restricted area that the hardly soluble drugs cannot be absorbed by oral administration, fully realize the medicinal effect, improve the oral bioavailability unprecedentedly, transform and upgrade thousands of hardly soluble drugs accounting for more than 40% of the total quantity of the drugs all around the world and provide a real technical support platform.

6. A precursor of the silicon dioxide aerogel is low in price and easy to obtain, has been widely applied in the drugs and foods, is a silicon-based medicinal and edible excipient which is in line with national and internal standards and has been used for many years and is also one of the excipients recorded in *Handbook of Pharmaceutical Excipients*, so that the safety of the silicon dioxide aerogel as the pharmaceutical excipient is reliable.

7. Anti-tumor oral drugs with high efficiency, low toxicity, economy and a "targeting function" can be prepared by applying the silicon dioxide aerogel to the field of preparation of the anti-tumor drugs, thereby solving the international pharmaceutical difficult problems of low bioavailability, high toxicity and side effects, poor curative effect and high treatment cost of anti-tumor clinical first-line and second-line drugs taking injections as the main dosage form, which have not been solved at home and aboard after decades of efforts. For example, in the oral dosage form of paclitaxel, a harmful solvent, namely polyoxyethylenated castor oil or Tween 80 and ethanol, which are currently adopted for solubilizing in the clinical first-line injection dosage form, do not need to be used, so that the bioavailability of patent drugs is improved unprecedentedly and the toxicity is greatly reduced. The nano-EPR (high-permeability and high-retention) effect which is highly respected by the international academic community is firstly really realized in the pharmaceutical practice, the bioavailability which can replace the injection with oral administration is firstly directly realized at the material level, and the nano-target delivery to a tumor part is simultaneously realized, so that original systemic toxicity and cytotoxic drugs with serious adverse reactions (such as paclitaxel, docetaxel and the like) can be aggregated to the tumor part, the curative effect is improved, the systemic toxicity and side effects are reduced, and a solid foundation is thus laid for taking the silicon dioxide aerogel as a platform material of a nano-target drug carrying system.

8. By combining the silicon dioxide aerogel with the drugs with the target treatment function (such as Xeloda, Iressa and the like), the "double-target" and "multi-target" anti-tumor applications can be realized, the "nano-target drug carrying system" is realized in a real sense and the dream of the researchers in the field of nano-drugs in half a century is realized (the nano-concept is proposed in 1940s).

9. In preclinical studies, the inventor makes a lot of experimental studies according to the requirements of related technical guideline of the anti-tumor drugs, and the research results prove that, in the studies taking human transplanted tumors as objects, the tumor inhibition rate of the oral nano preparations can achieve 80%, the absolute bioavailability exceeds 20%, and the toxicity is far lower than that of similar anti-tumor injection drugs in the comparison studies.

10. As the invention adopts the novel nano-material, namely the silicon dioxide aerogel as the drug carrying platform and fully uses the ultralarge specific surface area, ultralarge nano-drug carrying space, chemical and physical stability, biological inertia and other characteristics of the silicon dioxide aerogel, any special process methods and special equipment do not need to be adopted in the manufacturing process of the drugs, the standardized large-scale production of a variety of nano-drugs with stable quality can be achieved by using the common equipment for manufacturing the drugs, such as a homogenizer, a spray drying device and other equipment. Thus, the invention is expected to cause a revolution in the field of pharmacy or new preparation production ways.

The pharmaceutical action of nanosized drugs prepared by taking the silicon dioxide aerogel as the carrier material are illustrated through experiments by taking paclitaxel and docetaxel as examples, and the silicon dioxide aerogel used in the experiments was selected from the silicon dioxide aerogel with the following properties: the porosity was 95-99%, the aperture was 10-50nm, the specific surface area was 200-1000m$^2$/g, the density was 3-300kg/m$^3$ and the diameter of colloidal particles constituting a network was 1-50nm.

I. Research of bioavailability of nanosized paclitaxel oral dosage form of the invention in rat bodies

Purpose: the bioavailability research result of the preparation is the final standard for evaluating the preparation, and in the experiment, a clinical paclitaxel injection was used as a reference preparation, the bioavailability of a paclitaxel oral drug delivery system taking the silicon dioxide aerogel as a basic excipient in the rat bodies was investigated by detecting the concentration of paclitaxel in plasma of rats, and the experiment was intended to research whether the nano-paclitaxel oral dosage form can promote oral absorption of paclitaxel or not.

1. Materials and instruments

Paclitaxel active pharmaceutical ingredient (Yunnan Hande Pharmaceutical Co., Ltd.); paclitaxel injection (Huangshi Feiyun Pharmaceutical Co., Ltd.); nanosized paclitaxel (embodiment 1); methanol (chromatographic grade); acetonitrile (chromatographic grade); Diazepam (DZP, National Institute for Control of Biological Products); other reagents were analytically pure; and 15 healthy male SD rats with the body weight of $(210\pm20)$g, Guangdong Medical Experimental Animal Center. High performance liquid chromatograph (Dalian Elite Company); whirlpool mixer; table type high-speed centrifuge; electronic analytical balance; high-speed homogenizer (Shanghai Sower Instrument Co., Ltd.); ultrasonic cleaner; and table type centrifuge.

2. Experimental method

2.1 Establishment of method for determining paclitaxel in plasma

2.1.1 Chromatographic conditions

Chromatographic conditions: Elite SinoChrom 300A ODS-AP 5$\mu$m 4.6$\times$250mm
Mobile phase: methanol: water: acetonitrile=23: 41: 36; flow rate: 1.0ml/min; detection wavelength: 227nm; and sample size: 20$\mu$l.

2.1.2 Preparation of standard solution

Preparation of a paclitaxel stock solution: precisely weighing 10mg of paclitaxel, putting into a 50ml measuring flask, adding acetonitrile, dissolving, diluting to a scale and shaking up to obtain 200µg/ml of paclitaxel stock solution. An appropriate amount of the stock solution was precisely weighed, gradually diluted with methanol to a series of paclitaxel standard solutions of 2.5, 5.0, 10.0, 20.0 and 40.0µg/ml and preserved in a refrigerator at 4°C for later use.

Preparation of an internal standard solution: weighing about 10mg of a diazepam control product, precisely weighing, putting into a 100ml measuring flask, dissolving with methanol, diluting to the scale and shaking up to obtain an internal standard stock solution with the concentration of 100µg/ml. An appropriate amount of the stock solution was precisely weighed, diluted with methanol to prepare 10µg/ml of internal standard control product solution and preserved in the refrigerator at 4°C for later use.

2.1.3 Processing of blood sample test product

100ul of plasma sample was taken and put into an EP tube, 5µl (10µg/ml) and 40µl of $NaHCO_3$(1mol/L) was added, whirling was performed for 1min, 1ml of an extraction solvent, namely ethyl ether, was added, whirling was performed for 2min, centrifugation was performed at 3500r/min for 10min, supernatant fluid was taken and blow-dried under air flow of 40°C, the residue was dissolved in 40µl of mobile phase, whirling was performed for 1min, centrifugation was performed at 3500r/min for 10min, and then 20µl of supernatant fluid was taken for sample injection and analysis.

2.1.4 Preparation of standard curve

100ul of blank rat plasma was taken, the paclitaxel solutions with a series of concentrations were added to prepare paclitaxel plasma standard samples with the mass concentrations of 2.5, 5.0, 10.0, 20.0 and 40.0µg/ml, and the other operations were performed according to a method under the "pretreatment of plasma samples" to prepare a standard curve.

2.2 Research of bioavailability in rat bodies

12 healthy male SD rats were taken and randomly divided into four groups with three rats per group. Apaclitaxel injection preparation of 10mg/kg was injected into the tail vein of each rat in the first group; a nanosized paclitaxel suspension of 40mg/kg was used for performing one-time intragastric administration on each rat in the second group; a paclitaxel active pharmaceutical ingredient solution of 40mg/kg was used for performing on-time intragastric administration on each rat in the third group; and the fourth group was a blank serum group, and blood collection was performed at 1, 3, 6, 8 and 24h after drug administration. 0.5 ml of blood was taken every time and placed in the EP tube coated with heparin, and plasma was immediately subjected to centrifugal separation and put into the refrigerator at the temperature of -20°C for cryopreservation so as to be used for later test.

3. Results and discussions

3.1 Establishment of method for determining paclitaxel in plasma

3.1.1 Investigation of specificity of method

Blank plasma, blank plasma+diazepam, blank plasma+paclitaxel and a biological sample to be tested were processed according to the steps in item 2.1.3 and then tested, and the HPLC determination results showed that the retention time of paclitaxel was about 12.0min, the retention time of an internal standard substance was about 9min, the chromatographic peak separation was good and no impure peaks interfered with the determination.

3.1.2 Standard curve and linear range

Linear regression was performed on the concentration of paclitaxel, namely C(X) according to the peak area ratio of paclitaxel to diazepam, namely Atax/Adap(Y) to prepare a standard curve of content of paclitaxel in plasma, wherein the standard curve was as follows: Y=0.5136X+0.3, R2 =0.9998. The results showed that the concentration of paclitaxel was in the range of 2.5-40.0ug/ml, and the ratio of paclitaxel to diazepam, namely Atax/Adap(Y) had a good linear relationship with the concentration of paclitaxel, namely C(X).

3.2 Research of bioavailability in rat bodies

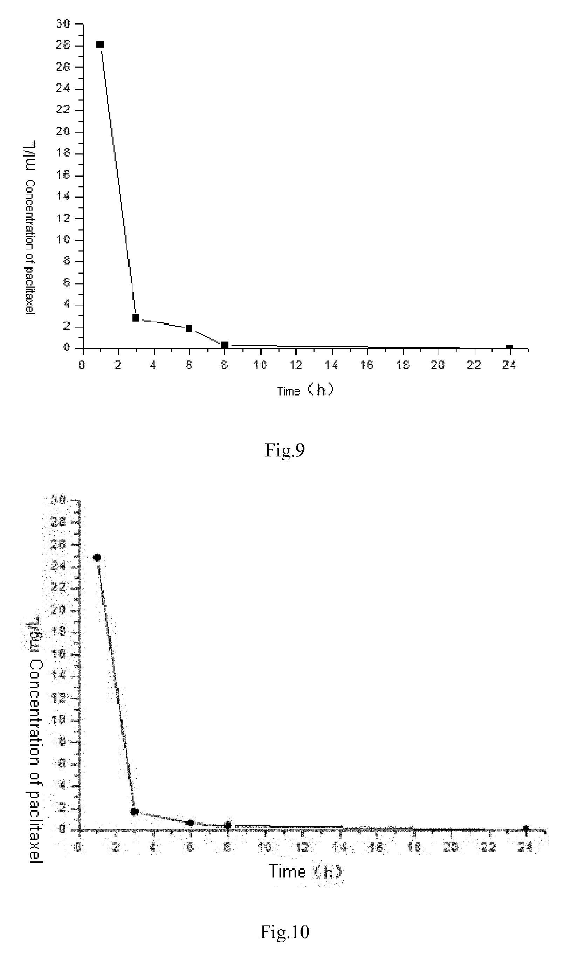

The average plasma concentration-time curves after drug administration by intragastric administration and intravenous injection of paclitaxel suspension in the rats were as shown in Fig. 9-Fig. 11, and the absolute bioavailability of paclitaxel nanoparticles was calculated according to the following formulas.

$$F(\%)=(\text{AUC oral administration}\times\text{intravenous injection dose})/(\text{AUC intravenous injection}\times\text{oral administration dose})\times100\%$$

$$F1=(34.275\text{x}10\text{mg/kg})/(42.34\text{x}40\text{mg/kg})\text{x}100\%=20.24\% \text{ (nano-paclitaxel)}$$

$$F2=(4.89\text{x}10\text{mg/kg})/(42.34\text{x}40\text{mg/kg})\text{x}l00\%=2.89\% \text{ (paclitaxel active pharmaceutical ingredient)}$$

The oral bioavailability of the paclitaxel active pharmaceutical ingredient was only 2.89%, and the absolute bioavailability of the nanosized paclitaxel oral drug delivery system was 20.24%, indicating that the silicon dioxide aerogel drug carrying system could significantly improve the bioavailability of oral drug administration of the hardly soluble drug, namely paclitaxel, and promote the absorption. The experimental results showed that the silicon dioxide aerogel+paclitaxel oral drug delivery system could greatly improve the oral bioavailability of paclitaxel.

II. Anti-tumor nude mouse experiment of nanosized paclitaxel of the invention

1. Materials: Balb/c female nude mice with a body weight of (18±2)g, purchased from Beijing Weitong Lihua Experimental Animal Technical Co., Ltd.; paclitaxel injection solution for experiment, purchased from Huangshi Feiyun Pharmaceutical Co., Ltd. (code number approved by SFDA: H20056466); and nano-paclitaxel for experiment was dry powder obtained in embodiment 1 of the invention.

2. Establishment of an animal model: collecting a sufficient amount of tumor cells, resuspending in a centrifugal tube with PBS and subcutaneously inoculating into the back of each nude mouse at every point according to $2\times10^6$ cells/0.1ml.

3. Experimental grouping and drug administration scheme: after the tumor model was established, grouping was performed according to 5 mice/group when the tumor diameter of each nude mouse was 4-6mm. The drug administration scheme was determined according to the oral bioavailability of 20%-30% by referring to the using method and using amount in a commercial drug instruction, related literature of latest *Handbook of Clinical Tumor Internal Medicine* and previous experimental results; a blank group (the blank group was only one and used as reference for each group); a pacitaxel injection group: intraperitoneal injection was performed once every three days; a paclitaxel active pharmaceutical ingredient group: oral intragastric administration was performed once for drug administration per day; and a nano-paclitaxel group: oral intragastric administration was performed once for drug administration per day.

4. Detection method: after drug administration, animals were raised normally, the general states of the animals were observed per day and the body weight of each animal was recorded. The tumor diameter was measured twice per week (by using a vernier caliper) and the tumor volume was calculated according to (v): $v=(ab^2)/2$ (in the formula, a was the long diameter of the tumor, and b was the short diameter of the tumor). The comparison of relative tumor volume (RTV) in each group was performed, and RTV=$v_t/v_0$, in the formula, $v_0$ was the tumor volume obtained by measurement in the day of performing caging and drug administration (Day0), and $v_t$ was the tumor volume which was measured every time; and the relative tumor volume was used for calculating the volume inhibition rate (VIR) of the drug against the tumor according to VIR=(1-RTV treatment group/RTV negative control group)×100%.

5. Experimental results

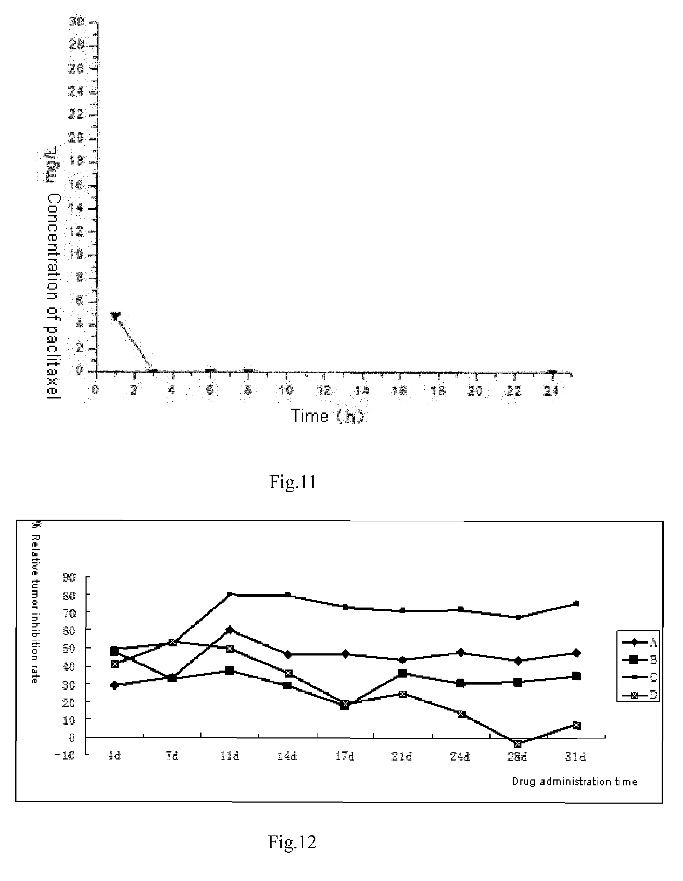

5.1 The experimental results of paclitaxel in treatment of human hepatoma BEL-7402 transplanted into the nude mice are as shown in the following table and Fig. 12.

[Table 1] - Relative tumor inhibition rate %

| | Dose | Time | 4d | 7d | 11d | 14d | 17d | 21d | 24d | 28d | 31d |
|---|---|---|---|---|---|---|---|---|---|---|---|
| Oral administration of nano-paclitaxel | 40mg/kg | A | 28.91 | 33.65 | 60.15 | 46.54 | 46.9 | 43.64 | 47.94 | 42.97 | 47.82 |
| | 80mg/kg | B | 47.75 | 32.88 | 37.28 | 29 | 17.58 | 35.99 | 30.18 | 31.08 | 34.57 |
| | 160mg/kg | C | 49.09 | 52.71 | 80.27 | 79.47 | 72.91 | 71.03 | 71.52 | 67.64 | 75.14 |
| Paclitaxel injection | 2mg/kg | D | 40.54 | 53.46 | 49.85 | 35.93 | 18.76 | 24.83 | 13.47 | -3.55 | 7.57 |

Note: the oral administration of nano-paclitaxel was performed according to 40mg/kg continuously for 14 days, the drug administration was stopped for 10 days, then the drug administration was performed for another 5 days, one mouse was dead and the grouping was performed according to 5 mice/group; the oral administration of the nano-paclitaxel was performed according to 80mg/kg continuously for 14 days, the drug administration was stopped for 10 days, then the drug administration was stopped for another 5 days, one mouse was dead and the grouping was performed according to 5 mice/group; the oral administration of the nano-paclitaxel was performed according to 160mg/kg continuously for 14 days, the drug administration was stopped for 10 days, and then the drug administration was performed for another 5 days, one mouse was dead, and the grouping was performed according to 5 mice/group; and the injection of the paclitaxel injection was performed according to 2mg/kg continuously for 14 days, the drug administration was performed for 10 days, the drug administration was performed for another 5 days, and the grouping was performed according to 5 mice/group.

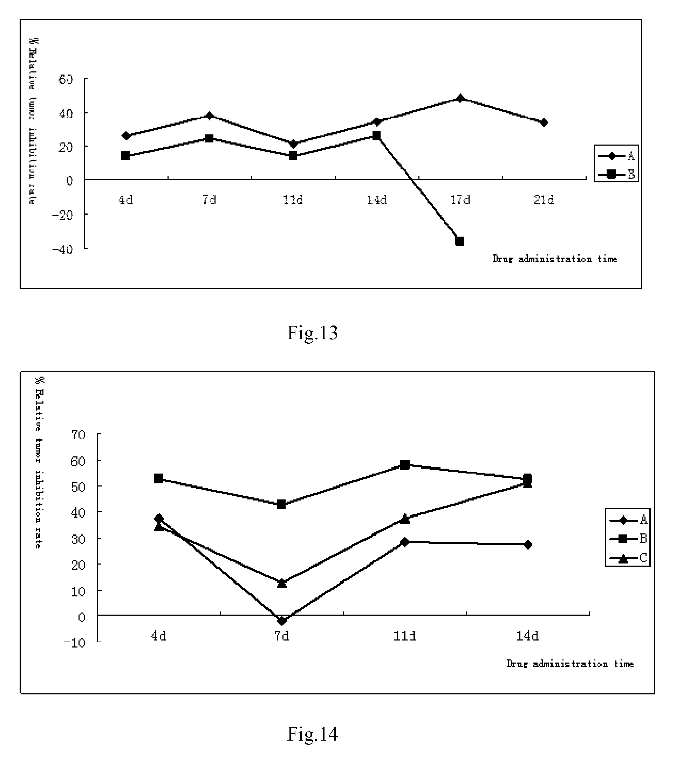

5.2 The experimental results of paclitaxel in the treatment of human lung cancer NCI-1299 transplanted into the nude mice are as shown in the following table and Fig. 13.

[Table 2]

| Relative tumor inhibition rate % | | | | | | | | |
|---|---|---|---|---|---|---|---|---|
| | Dose | Time | 4d | 7d | 11d | 14d | 17d | 21d |
| Oral administration of nano-paclitaxel | 80mg/kg | A | 26.1 | 37.81 | 21.33 | 34.14 | 48.4 | 33.78 |
| Paclitaxel injection | 2mg/kg | B | 13.98 | 24.44 | 14.16 | 26.06 | -35.75 | |
| Note: the oral administration of the nano-paclitaxel was performed according to 80mg/kg, one mouse was dead and the grouping was performed according to 5 mice/group; and the injection was performed according 2mg/kg in the paclitaxel injection solution group continuously for drug administration for 4 days, the mice had ascites in succession, all of the mice were dead on the 17th day, and the grouping was performed according to 5 mice/group. | | | | | | | | |

5.3 The experimental results of paclitaxel in the treatment of human breast cancer MCF-7 transplanted into the nude mice are as shown in the following table and Fig. 14.

[Table 3]

| Relative tumor inhibition rate % | | | | | | |
|---|---|---|---|---|---|---|
| | Dose | Time | 4d | 7d | 11d | 14d |
| Oral administration of nano-paclitaxel | 80mg/kg | A | 37.66 | -1.81 | 28.37 | 27.47 |
| | 160mg/kg | B | 52.46 | 42.7 | 58.28 | 52.42 |
| Paclitaxel injection | 10mg/kg | C | 34.37 | 12.7 | 37.57 | 50.99 |
| Note: the oral administration of nano-paclitaxel was performed according to 80mg/kg continuously for 14 days, no death was found, and the grouping was performed according to 5 mice/group; the oral administration of the nano-paclitaxel was performed according to 160mg/kg continuously for 14 days, no death was found and the grouping was performed according to 5 mice/group; and the injection was performed in the paclitaxel injection solution group according to 10mg/kg once every three days, the drug administration was performed for 5 times in total, and no death was found. | | | | | | |

5.4 Results and discussions

1. In the experiments, according to the characteristic of using as much anti-tumor drug as possible to fast kill cancer cells, the using amount of the drug was designed according to the maximum tolerance degree (MTD) to enable the anti-cancer effect of a positive control commercial drug to achieve the best level, the commercial drug and the oral nano-drug of the invention were investigated in the aspect of safety while the anti-cancer effects of the two were compared, and the determination of the treatment dose in the nude mice bearing the tumors transplanted from human was the most direct method for researching and investigating the safety of the anti-tumor drug before clinical use;

2. Three types of human transplanted tumor cells were used respectively for performing tumor inhibition contrast test on the commercial paclitaxel injection solution and the oral nano-paclitaxel of the invention, and the results were that the death rate of the commercial paclitaxel injection solution group was higher than that of the oral nano-paclitaxel group, and the treatment effect was lower than that of the oral group; and

3. The experimental results showed that the relative tumor inhibition rate of the oral nano-preparation of the invention was better than the level of the commercial injection drug, and the safety was also better than that of the commercial drug, prompting that the oral nano-drug of the invention had good effects of improving the quality of life of patients and prolonging the survival time.

III. Pharmacological experiments entrusted to Nanjing Kaiji Biotechnology Development Co., Ltd.

1. Experimental purpose:

Test samples were tested according to the requirements of *Guide Principles of Pharmacodynamics of Anti-Tumor Drugs* and *Guide Principles of Non-Clinical Research Technology of Cytotoxic Anti-Tumor Drugs* to judge whether the test samples had an inhibition action against the growth of human lung cancer cell A549 nude mice xenograft tumors or not and action strength.

2. Test samples:

Paclitaxel injection solution: Sichuan Taiji Group Co., Ltd., batch number: 12100031 and specification: 30mg/5ml. When in use, the paclitaxel injection solution was diluted with physiological saline to the required concentration. Docetaxel injection solution: Zhejiang Wanma Pharmaceutical Co., Ltd., batch number: H20051044 and specification: 20mg/0.5ml. Before the use, the docetaxel injection solution was firstly diluted with 2ml of dilution solution, and when in use, the docetaxel injection solution was diluted with the physiological saline to the required concentration. Nano-paclitaxel and nano-docetaxel: dry powder obtained in embodiment 1 and embodiment 3 of the invention was used respectively, the dry powder was used instantly after weighing and preparation, the dry powder was weighed by an analytical balance, then distilled water was added, ultrasonic dissolution was performed to form the suspension, and then intragastric administration was performed for drug administration.

3. Test animals:

Source, germline and strain: BALB/c nude mice, provided by the Experimental Animal Center of
Chinese Academy of Military Medical Sciences. Production license of experimental animals: SCXK (Army) 2007-004
Certificate number: 0001015
Use license of experimental animals: SYXK (Su) 2012-010, age of days: 4-5w; body weight: 18-22g; sex: male;
number of animals: 6 mice/group, and 54 mice in total.

4. Groups and drug administration schemes are as shown in the following table.

[Table 4]

| Group | Drug administration scheme | | | |
|---|---|---|---|---|
| | Drug administration way | Drug administration dose (mg/Kg) | Drug administration period | Drug administration frequency |
| Model control group | Oral administration by intragastric administration | Physiological saline | - | 1 day/time |
| Commercial paclitaxel | Intraperitoneal injection | 10 mg/kg | 2 weeks | 3 days/time |
| Commercial docetaxel | Intraperitoneal injection | 10 mg/kg | 2 weeks | 3 days/time |
| Self-made nano-paclitaxel | Oral administration by intragastric administration | 50 mg/kg | 2 weeks | Every day |
| | | 100 mg/kg | | |
| | | 200 mg/kg | | |
| Self-made nano-docetaxel | Oral administration by intragastric administration | 50 mg/kg | 2 weeks | Every day |
| | | 100 mg/kg | | |
| | | 200 mg/kg | | |

5. Experimental method

5.1 Preparation of model

A cultured human lung cancer A549 suspension was collected, the concentration was $1 \times 10^7$/ml, and the suspension was inoculated subcutaneously to the right armpit of each nude mouse according to 0.1ml/mouse.

5.2 Grouping and drug administration

The diameter of the transplanted tumor in each nude mouse was measured by using the vernier caliper, and when the tumors grew to 50-75mm$^3$ after 11 days of inoculation, the animals were randomly grouped according to 6 mice/group. Meanwhile, the drug administration was started to perform on the nude mice in each group, for the drug administration schemes and the groups, please refer to the drug administration schemes, and the anti-tumor effect of the test samples were dynamically observed by using the method of measuring the tumor diameter. After the drug administration, the mice were killed and the tumor blocks were surgically stripped for weighing.

5.3 Observation indexes

The calculation formula of the tumor volume (TV) was as follows: $TV=1/2 \times a \times b^2$, wherein a and b respectively represented length and width.
The relative tumor volume (RTV) was calculated according to the measurement result, and the calculation formula was as follows:

$$RTV=V_t/V_0,$$ wherein $V_0$ was the tumor volume obtained by measurement during caging and drug administration (d0) and $V_t$ was the tumor volume which was measured every time.

The evaluation index of anti-tumor activity was relative tumor proliferation rate T/C (%), and the calculation formula was as follows:

$$T/C\ (\%)=T_{rtv}/C_{rtv} \times 100,$$ wherein $T_{rtv}$ was the RTV of the treatment group; and $C_{rtv}$ was the RTV of the model group.

The evaluation index of anti-tumor activity was tumor growth inhibition rate (%), and the calculation formula was as follows:

$$\text{Tumor growth inhibition rate} = (\text{average tumor weight of model group} - \text{average tumor weight of drug administration group})/\text{average tumor weight of model group} \times 100\%.$$

5.4 Statistical processing

The mean value was represented by $X \pm SD$, the analysis between groups used t test to perform statistical processing, and SPSS (Staffstical Package for the Social Science) 17.0 was used to perform statistical analysis on the results.

6. Experimental results

Affects of test samples on body weight of nude mice with human lung cancer cell A549 xenograft tumors ($X \pm SD$, n=6, and unit: g)

[Table 5]

| Group/number of times | First time | Second time | Third time | Fourth time | Fifth time | Sixth time | Seventh time | Eighth time |
|---|---|---|---|---|---|---|---|---|
| Model control group | 19.4±0.9 | 20.7±1.1 | 21.2±1.2 | 22.1±1.1 | 22.5±1.1 | 23.3±1.5 | 23.5±1.4 | 24.4±1.6 |
| Commercial paclitaxel | 19.7±1.3 | 21.6±1.4 | 22.0±1.5 | 22.8±1.5 | 22.2±1.6 | 22.3±1.8 | 22.7±1.9 | 23.6±1.4 |
| Commercial docetaxel | 21.6±0.9 | 22.4±0.9 | 22.6±1.1 | 23.2±1.3 | 23.1±1.3 | 22.1±2.3 | 23.0±2.5 | 22.5±2.6 |
| Nano-paclitaxel-50 | 19.8±1.7 | 21.9±1.3 | 21.4±3.1 | 21.9±2.8 | 22.3±2.4 | 22.4±1.9 | 23.2±1.1 | 23.4±1.0 |
| Nano-paclitaxel-100 | 18.7±2.5 | 19.7±2.4 | 20.7±2.5 | 21.4±2.5 | 21.6±2.4 | 21.5±2.1 | 22.1±2.4 | 22.1±2.4 |
| Nano-paclitaxel-200 | 19.3±2.1 | 20.7±2.2 | 21.1±2.1 | 21.8±1.8 | 22.3±1.8 | 22.7±1.8 | 23.4±1.9 | 23.9±1.8 |
| Nano-docetaxel-50 | 20.7±1.0 | 22.1±1.0 | 22.5±1.0 | 23.2±0.9 | 23.5±1.1 | 23.7±1.3 | 24.1±1.5 | 24.5±1.5 |
| Nano-docetaxel-100 | 121.5±0.9 | 23.0±1.0 | 23.5±0.9 | 24.1±0.6 | 24.1±0.4 | 24.8±0.7 | 25.2±0.8 | 25.4±0.8 |
| Nano-docetaxel-200 | 120.8±0.9 | 21.8±1.0 | 22.2±0.9 | 22.6±0.7 | 23.0±0.9 | 23.4±1.1 | 24.1±1.8 | 24.5±1.4 |

Affects of test samples on changes in growth volume of human lung cancer cell A549 xenograft tumors in the nude mice (X±SD, n=6, and unit: cm$^3$)

[Table 6]

| Group/number of times | First time | Second time | | |
|---|---|---|---|---|
| | Tumor volume | Tumor volume | RTV | T/C |
| Model control group | 0.050±0.008 | 0.059±0.015 | 1.189±0.256 | - |
| Commercial paclitaxel-10mg/kg | 0.052±0.009 | 0.047±0.017 | 0.925±0.323 | 77.74% |
| Commercial docetaxel-10mg/kg | 0.051±0.013 | 0.048±0.024 | 0.930±0.305 | 78.18% |
| Nano-paclitaxel-50mg/kg | 0.053±0.016 | 0.056±0.026 | 1.047±0.262 | 88.05% |
| Nano-paclitaxel-100mg/kg | 0.052±0.017 | 0.051±0.021 | 0.966±0.226 | 81.19% |
| Nano-paclitaxel-200mg/kg | 0.055±0.011 | 0.052±0.012 | 0.972±0.221 | 81.70% |
| Nano-docetaxel-50mg/kg | 0.050±0.008 | 0.053±0.008 | 1.056±0.144 | 88.78% |
| Nano-docetaxel-100mg/kg | 0.051±0.011 | 0.044±0.009 | 0.882±0.152 | 74.21% |
| Nano-docetaxel-200mg/kg | 0.054±0.006 | 0.049±0.012 | 0.917±0.215 | 77.08% |

[Table 7]

| Third time | | | Fourth time | | |
|---|---|---|---|---|---|
| Tumor volume | RTV | T/C | Tumor volume | RTV | T/C |
| 0.075±0.016 | 1.524±0.297 | - | 0.102±0.025 | 2.066±0.554 | - |
| 0.047±0.014 | 0.939±0.329 | 61.63% | 0.053±0.015 | 1.056±0.326 | 51.11% |
| 0.045±0.012 | 0.911±0.253 | 59.81% | 0.044±0.022 | 0.907±0.540 | 43.90% |
| 0.064±0.011 | 1.330±0.561 | 87.28% | 0.065±0.025 | 1.304±0.582 | 63.14% |
| 0.063±0.017 | 1.246±0.425 | 81.80% | 0.068±0.015 | 1.371±0.447 | 66.38% |
| 0.064±0.015 | 1.192±0.290 | 78.25% | 0.064±0.023 | 1.186±0.410 | 57.41% |
| 0.057±0.011 | 1.137±0.209 | 74.61% | 0.069±0.025 | 1.348±0.360 | 65.26% |
| 0.050±0.023 | 0.980±0.348 | 64.34% | 0.067±0.030 | 1.348±0.541 | 65.23% |
| 0.052±0.018 | 0.975±0.372 | 64.01% | 0.063±0.025 | 1.181±0.481 | 57.14% |

[Table 8]

| Fifth time | | | Sixth time | | |
|---|---|---|---|---|---|
| Tumor volume | RTV | T/C | Tumor volume | RTV | T/C |
| 0.132±0.037 | 2.665±0.652 | - | 0.180±0.035 | 3.643±0.609 | - |
| 0.059±0.020 | 1.134±0.236 | 42.55% | 0.063±0.017 | 1.243±0.337 | 34.11% |
| 0.055±0.024 | 1.113±0.559 | 41.75% | 0.058±0.020 | 1.181±0.539 | 32.61% |
| 0.076±0.016 | 1.549±0.569 | 58.14% | 0.096±0.026 | 1.916±0.808 | 54.24% |
| 0.085±0.017 | 1.723±0.512 | 64.64% | 0.097±0.029 | 1.997±0.912 | 54.81% |
| 0.076±0.022 | 1.423±0.424 | 53.39% | 0.106±0.035 | 2.018±0.763 | 55.38% |
| 0.067±0.018 | 1.323±0.296 | 49.66% | 0.084±0.027 | 1.660±0.449 | 45.56% |
| 0.077±0.037 | 1.545±0.724 | 57.97% | 0.085±0.033 | 1.713±0.646 | 47.02% |

(continued)

| Fifth time | | | Sixth time | | |
|---|---|---|---|---|---|
| Tumor volume | RTV | T/C | Tumor volume | RTV | T/C |
| 0.073±0.027 | 1.372±0.534 | 51.48% | 0.091±0.030 | 1.704±0.615 | 46.77% |

[Table 9]

| Seventh time | | | Eighth time | | |
|---|---|---|---|---|---|
| Tumor volume | RTV | T/C | Tumor volume | RTV | T/C |
| 0.232±0.061 | 4.672±1.045 | - | 0.428±0.117 | 8.083±2.260 | - |
| 0.081±0.050 | 1.559±0.807 | 33.37% | 0.101±0.046 | 2.031±1.031 | 23.39% |
| 0.063±0.019 | 1.262±0.395 | 27.02% | 0.079±0.021 | 1.575±0.324 | 18.14% |
| 0.118±0.033 | 2.414±1.053 | 51.68% | 0.174±0.033 | 3.539±1.165 | 40.76% |
| 0.113±0.052 | 2.418±1.652 | 51.75% | 0.163±0.082 | 3.473±2.489 | 40.00% |
| 0.133±0.041 | 2.476±0.710 | 53.01% | 0.185±0.058 | 3.456±1.030 | 39.80% |
| 0.108±0.051 | 2.125±0.958 | 45.49% | 0.154±0.045 | 3.076±0.882 | 35.42% |
| 0.109±0.027 | 2.179±0.436 | 46.64% | 0.153±0.051 | 3.050±0.965 | 35.12% |
| 0.113±0.040 | 2.130±0.860 | 45.60% | 0.146±0.041 | 3.739±0.905 | 31.55% |

Inhibition effects of test samples on the growth of human lung cancer cell A549 xenograft tumors in the nude mice (X±SD, n=6)

[Table 10]

| Group | Number of animals | Body weight of animal (g) | Tumor volume (cm³) | Drug administration scheme | | | | Experimental period |
|---|---|---|---|---|---|---|---|---|
| | Initial | Initial | Initial | Drug administration way | Drug administration dose | Number of drug administration times | Drug administration frequency | (day) |
| Model control group | 16 | 19.4±+ 0.9 | 0.050±+0.008 | Oral intragastric administration | - | 14 | Oral administration per day | 25 |
| Commercial paclitaxel-10mg/kg | 6 | 19.7±+ 1.3 | 0.052±+0.009 | Intraperitoneal injection | 10mg/kg | 5 | Once every three days | 25 |
| Commercial docetaxel-10mg/kg | 6 | 21.6±+ 0.9 | 0.051±+0.013 | Intraperitoneal injection | 10mg/kg | 5 | Once every three days | 25 |
| Nano-paclitaxel-50 mg/kg | 6 | 19.8±+ 1.7 | 0.053±+0.016 | Oral intragastric administration | 50mg/kg | 14 | Oral administration per day | 25 |
| Nano-paclitaxel-100mg/kg | 6 | 18.7±+ 2.5 | 0.052±+0.017 | Oral intragastric administration | 100mg/kg | 14 | Oral administration per day | 25 |
| Nano-paclitaxel-200mg/kg | 6 | 19.3±+ 2.1 | 0.055±+0.011 | Oral intragastric administration | 200mg/kg | 14 | Oral administration per day | 25 |
| Nano-docetaxel-50 mg/kg | 6 | 20.7±+ 1.0 | 0.050±+0.008 | Oral intragastric administration | 50mg/k g | 14 | Oral administration per day | 25 |
| Nano-docetaxel-100 mg/kg | 6 | 21.5±+ 0.9 | 0.051±+0.011 | Oral intragastric administration | 100mg/kg | 14 | Oral administration per day | 25 |
| Nano-docetaxel-200 mg/kg | 6 | 20.8±+ 0.9 | 0.054±+0.006 | Oral intragastric administration | 200mg/ kg | 14 | Oral administration per day | 25 |

[Table 11]

| Group | Number of killed animals | Body weight of killed animal (g) | Tumor volume of killed animal ($cm^3$) | Relative tumor proliferation rate T/C | Tumor weight of killed animal (g) | Tumor inhibition rate |
|---|---|---|---|---|---|---|
| Model group | 6 | 24.4±+1.6 | 0.428±+0.117 | - | 0.33±0.15 | - |
| Commercial paclitaxel-10mg/kg | 6 | 23.6±+1.4 | 0.101±0.046 | 23.39% | 0.12±+0.06 | 62.81% |
| Commercial docetaxel-10mg/kg | 6 | 22.5±+2.6 | 0.079±+0.021 | 18.14% | 0.07±+0.03 | 78.89% |
| Nano-paclitaxel-50mg/kg | 6 | 23.4±+1.0 | 0.174±+0.033 | 40.76% | 0.22±+0.07. | 33.67 |
| Nano-paclitaxel-100mg/kg | 6 | 22.1±2.4 | 0.163±+0.082 | 40.00% | 0.19±+0.064 | 2.21 |
| Nano-paclitaxel-200mg/kg | 6 | 23.9±+1.8 | 0.185±+0.058 | 39.80% | 0.22±+0.09. | 34.67 |
| Nano-docetaxel-50mg/kg | 6 | 24.5±+1.5 | 0.154±+0.045 | 35.42% | 0.17±+0.07 | 49.75 |
| Nano-docetaxel-100mg/kg | 6 | 25.4±+0.8 | 0.153±+0.051 | 35.12% | 0.10±+0.02 | 69.85 |
| Nano-docetaxel-200mg/kg | 6 | 24.5±+1.4 | 0.146±+0.041 | 31.55% | 0.07±+0.05 | 78.89 |

7. Discussions:

The experiments established the human lung cancer A549 nude mouse xenograft tumor model, and the model was used for evaluating the anti-tumor activity of the test samples, namely nano-paclitaxel and nano-docetaxel. The experimental results were as follows: the tumor inhibition rate of the test sample, namely nano-paclitaxel in each group of the low dose group of 50mg/kg, the medium dose group of 100mg/kg and high dose group of 200mg/kg was 33.67%, 42.21% and 34.67% respectively. The tumor inhibition rate of the test sample, namely nano-docetaxel in each group of the low dose group of 50mg/kg, the medium dose group of 100mg/kg and high dose group of 200mg/kg was 49.75%, 69.85% and 78.89% respectively. While the tumor inhibition rate of each of the positive control groups, namely commercial paclitaxel and commercial docetaxel was 62.81% and 78.89% respectively. The conclusion was that the nano-paclitaxel had a certain anti-tumor effect, the nano-docetaxel had obvious anti-tumor effect, while the medium dose group and the high dose group had significant difference, namely P<0.01.

Brief Description of the Drawings

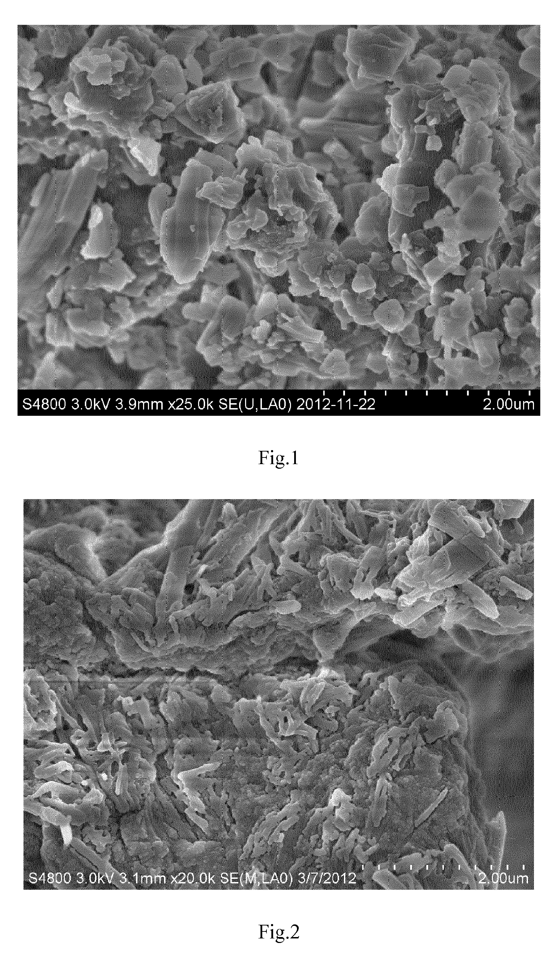

Fig. 1 is an electron microscope image of silicon dioxide aerogel of the invention;
Fig. 2 is an electron microscope image of a paclitaxel active pharmaceutical ingredient;

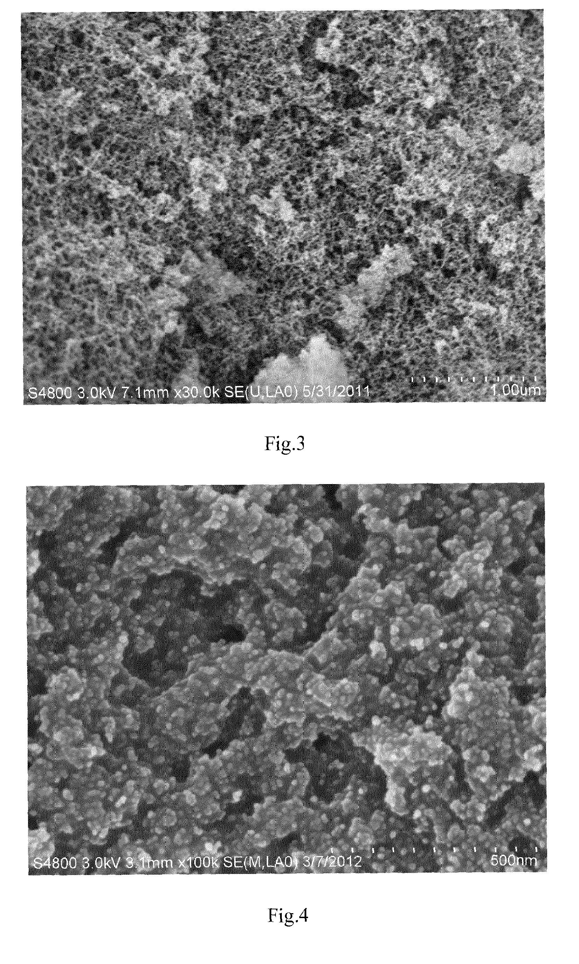

Fig. 3 is an electron microscope image of nanosized paclitaxel prepared by taking silicon dioxide aerogel as a carrier;
Fig. 4 is an electron microscope image of nanosized insulin prepared by taking silicon dioxide aerogel as a carrier;

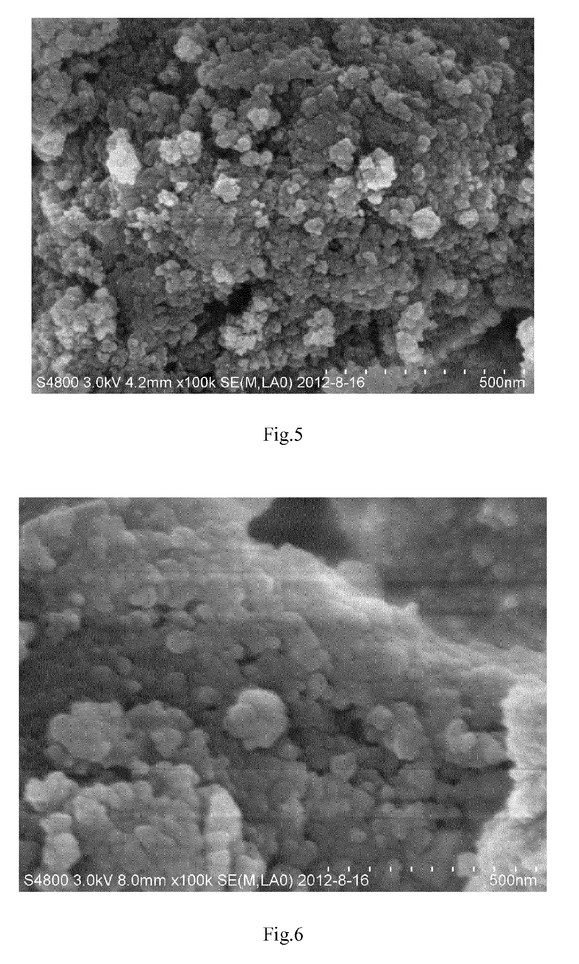

Fig. 5 is an electron microscope image of nanosized doxorubicin hydrochloride prepared by taking silicon dioxide aerogel as a carrier;
Fig. 6 is an electron microscope image of nanosized cisplatin prepared by taking silicon dioxide aerogel as a carrier;

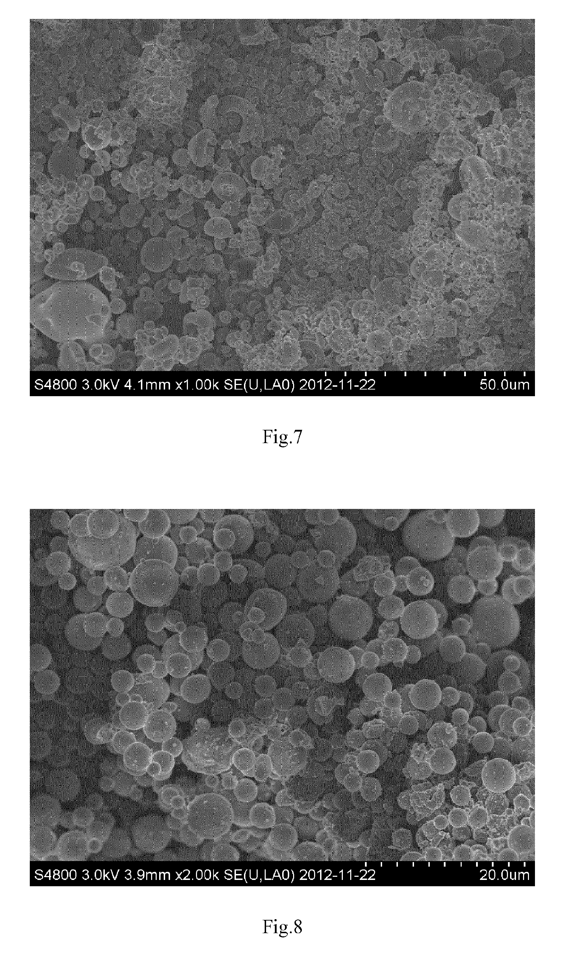

Fig. 7 is an electron microscope image of nanosized capecitabine prepared by taking silicon dioxide aerogel as a carrier;
Fig. 8 is an electron microscope image of nanosized cyclophosphamide prepared by taking silicon dioxide aerogel as a carrier;
Fig. 9 is a paclitaxel plasma drug concentration curve of a rat after tail vein injection of a paclitaxel injection preparation according to 10mg/kg;

Fig. 10 is a paclitaxel plasma drug concentration curve of a rat after one-time intragastric administration of a nano-paclitaxel oral suspension according to 40mg/kg;

Fig. 11 is a paclitaxel plasma drug concentration curve of a rat after one-time intragastric administration of a paclitaxel active pharmaceutical ingredient solution according to 40mg/kg;

Fig. 12 is relative tumor inhibition rate curve diagrams against human hepatoma BEL-7402 transplanted into nude mice in experimental research results of anti-tumor nude mice;

Fig. 13 is relative tumor inhibition rate curve diagrams against human non-small-cell carcinoma NCI-1299 transplanted into nude mice in experimental research results of anti-tumor nude mice;

and

Fig. 14 is relative tumor inhibition rate curve diagrams against human breast cancer MCF-7 transplanted into nude mice in experimental research results of anti-tumor nude mice.

Detailed Description of the Embodiments

The invention will be described in detail below with reference to the accompanying drawings and the following embodiments are used for explaining the invention rather than limiting the invention.

Embodiment 1

Preparation of paclitaxel nanoparticles:

1.1g of a paclitaxel active pharmaceutical ingredient (Yunnan Hande Pharmaceutical Co., Ltd.) was added into 20ml of anhydrous ethanol for dissolution;

2. 2g of silicon dioxide aerogel (with the porosity of 95%, the aperture of 10nm, the specific surface area of $1000m^2/g$, the density of $300kg/m^3$ and the diameter of colloidal particles constituting a network of 20nm) after heat treatment at the temperature of 300°C was added for adsorption;

3. Drying was performed in an oven at the temperature of 60°C after complete adsorption;

4. 100ml of pure water was added after drying, and then emulsification was performed by an ordinary emulsifier at 25000rpm/min for 5min;

5. Operation was performed in a high-pressure homogenizer (Shanghai Donghua GYB30-6S) at 400bar, cycling was performed for 6 times, and the operation was performed for 10min; and

6. Homogenate was spray-dried in an experimental spray-drying machine (Shanghai Shunyi Science and Technology SP-1500) under the parameters that the temperature was 130°C, the flow rate was 500ml/H and a spray head was 0.75mm, and drying was performed to obtain the paclitaxel nanoparticles.

Embodiment 2

Preparation of paclitaxel nanoparticles:

1.1g of a paclitaxel active pharmaceutical ingredient (Yunnan Hande Pharmaceutical Co., Ltd.) was added into 100ml of anhydrous ethanol for dissolution;

2. 10g of hydrophilic silicon dioxide aerogel (with the porosity of 97%, the aperture of 16nm, the specific surface area of $500m^2/g$, the density of $150kg/m^3$ and the diameter of colloidal particles constituting a network of 50nm) was added for adsorption;

3. Freeze-drying was performed after complete adsorption;

4. 110ml of pure water was added after drying, and then emulsification was performed by an ordinary emulsifier at 25000rpm/min for 5min;

5. Operation was performed in a high-pressure homogenizer (Shanghai Donghua GYB30-6S) at 400bar, cycling was performed for 7 times, and the operation was performed for 10min; and

6. Homogenate was spray-dried in an experimental spray-drying machine (Shanghai Shunyi Science and Technology SP-1500) under the parameters that the temperature was 130°C, the flow rate was 500ml/H and a spray head was 0.75mm, and drying was performed to obtain the paclitaxel nanoparticles.

Embodiment 3

Preparation of docetaxel nanoparticles:

1. 1g of a docetaxel active pharmaceutical ingredient (Shanghai Zhongxi Sunve Pharmaceutical Co., Ltd.) was added into 20ml of anhydrous ethanol for dissolution;
2. 2g of silicon dioxide aerogel (with the porosity of 98%, the aperture of 45nm, the specific surface area of $800m^2/g$, the density of $50kg/m^3$ and the diameter of colloidal particles constituting a network of 10nm) after heat treatment at the temperature of 500°C was added for adsorption;
3. Drying was performed in an oven at the temperature of 60°C after complete adsorption;
4. 100ml of pure water was added after drying, and then emulsification was performed by an ordinary emulsifier at 25000rpm/min for 5min;
5. Operation was performed in a high-pressure homogenizer (Shanghai Donghua GYB30-6S) at 400bar, cycling was performed for 6 times, and the operation was performed for 10min; and
6. Homogenate was spray-dried in an experimental spray-drying machine (Shanghai Shunyi Science and Technology SP-1500) under the parameters that the temperature was 130°C, the flow rate was 500ml/h and a spray head was 0.75mm, and drying was performed to obtain the docetaxel nanoparticles.

Embodiment 4

Preparation of insulin nanoparticles:

1. 1g of an insulin active pharmaceutical ingredient (Jiangsu Wanbang Biochemical Pharmaceutical Stock Co., Ltd.) was added into 150ml of 0.01mol/L AR grade hydrochloric acid for dissolution;
2. 15g of silicon dioxide aerogel (with the porosity of 99%, the aperture of 50nm, the specific surface area of $500m^2/g$, the density of $3kg/m^3$ and the diameter of colloidal particles constituting a network of 1nm) after heat treatment at the temperature of 1000°C was added for adsorption;
3. Drying was performed in a freeze-drying machine for 4h after complete adsorption;
4. Another 10g of PEG-600 was taken and added into 1000ml of anhydrous ethanol for dissolution;
5. A solid after freeze-drying in step 3 was added into the ethanol solution of PEG-600, and emulsification was performed by an ultrasonic emulsifier for 3min;
6. An emulsified solution obtained in step 5 was dried in an electric heating constant-temperature drying box at the temperature of 60°C for 12h; and
7. A solid after drying in step 6 was ground and screened by a 200-mesh screen to obtain the insulin nanoparticles.

Embodiment 5

Preparation of doxorubicin hydrochloride nanoparticles:

1. 1g of a doxorubicin hydrochloride active pharmaceutical ingredient (Wuhan Dahua Weiye Pharmaceutical Co., Ltd.) was added into 200ml of pure water for complete dissolution;
2. 20g of silicon dioxide aerogel (with the porosity of 98%, the aperture of 45nm, the specific surface area of $800m^2/g$, the density of $50kg/m^3$ and the diameter of colloidal particles constituting a network of 10nm) after heat treatment at the temperature of 500°C was added for adsorption;
3. Freeze-drying was performed after complete adsorption;
4. 200ml of pure water was added after drying, and then emulsification was performed by an ordinary emulsifier at 25000rpm/min for 5min;
5. Operation was performed in a high-pressure homogenizer (Shanghai Donghua GYB30-6S) at 400bar, cycling was performed for 7 times, and the operation was performed for 10min; and
6. Homogenate was spray-dried in an experimental spray-drying machine (Shanghai Shunyi Science and Technology SP-1500) under the parameters that the temperature was 130°C, the flow rate was 500ml/H and a spray head was 0.75mm, and drying was performed to obtain the doxorubicin hydrochloride nanoparticles.

Embodiment 6

Preparation of cisplatin nanoparticles:

1. 1g of a cisplatin active pharmaceutical ingredient (Shandong Boyuan Pharmaceutical Co., Ltd.) was added into 5ml of a 1.5% NaCl solution for dissolution;
2. 0.5g of silicon dioxide aerogel (with the porosity of 97%, the aperture of 34nm, the specific surface area of $200m^2/g$, the density of $120kg/m^3$ and the diameter of colloidal particles constituting a network of 25nm) after heat treatment at the temperature of 800°C was added for adsorption;
3. Drying was performed in an oven at the temperature of 60°C after complete adsorption;
4. 50ml of pure water was added after drying, and then emulsification was performed by an ordinary emulsifier at 25000rpm/min for 5min;
5. Operation was performed in a high-pressure homogenizer (Shanghai Donghua GYB30-6S) at 400bar, cycling was performed for 6 times, and the operation was performed for 10min; and
6. Homogenate was spray-dried in an experimental spray-drying machine (Shanghai Shunyi Science and Technology SP-1500) under the parameters that the temperature was 130°C, the flow rate was 500ml/H and a spray head was 0.75mm, and drying was performed to obtain the cisplatin nanoparticles.

Embodiment 7

Preparation of capecitabine nanoparticles:

1. 1g of a capecitabine active pharmaceutical ingredient (Jinan Fuchuang Pharmaceutical Science and Technology Co., Ltd.) was added into 20ml of anhydrous ethanol for dissolution;
2. 2g of silicon dioxide aerogel (with the porosity of 98%, the aperture of 40nm, the specific surface area of $400m^2/g$, the density of $100kg/m^3$ and the diameter of colloidal particles constituting a network of 5nm) after heat treatment at the temperature of 1000°C was added for adsorption;
3. Drying was performed in an oven at the temperature of 60°C after complete adsorption;
4. 100ml of pure water was added after drying, and then emulsification was performed by an ordinary emulsifier at 25000rpm/min for 5min;
5. Operation was performed in a high-pressure homogenizer (Shanghai Donghua GYB30-6S) at 400bar, cycling was performed for 6 times, and the operation was performed for 10min; and
6. Homogenate was spray-dried in an experimental spray-drying machine (Shanghai Shunyi Science and Technology SP-1500) under the parameters that the temperature was 130°C, the flow rate was 500ml/H and a spray head was 0.75mm, and drying was performed to obtain the nanosized capecitabine particles.

Embodiment 8

Preparation of cyclophosphamide nanoparticles:

1. 1g of a cyclophosphamide active pharmaceutical ingredient (Hubei Haiboyuan Chemical Industry Co., Ltd.) was added into 20ml of anhydrous ethanol for dissolution;
2. 2g of silicon dioxide aerogel (with the porosity of 99%, the aperture of 50nm, the specific surface area of $1000m^2/g$, the density of $3kg/m^3$ and the diameter of colloidal particles constituting a network of 1nm) after heat treatment at the temperature of 700°C was added for adsorption;
3. Freeze-drying was performed after complete adsorption;
4. Another 1g of PEG-4000 was taken and added into 200ml of anhydrous ethanol for dissolution;
5. A solid after freeze-drying in step 3 was added into the ethanol solution of PEG-4000, and emulsification was performed by an ultrasonic emulsifier for 3min;
6. An emulsified solution in step 5 was dried in an electric heating constant-temperature drying box at the temperature of 60°C for 12h; and
7. A solid after drying in step 6 was ground and screened by a 200-mesh screen to obtain the nanosized cyclophosphamide particles.

Embodiment 9

The nanoparticles obtained in each of the embodiments 1 to 8 were uniformly mixed with an appropriate amount of microcrystalline cellulose, starch and magnesium stearate and subjected to tablet pressing by a tablet pressing machine for preparing tablets.

Embodiment 10

The nanoparticles obtained in each of the embodiments 1 to 8 were directly loaded into hard capsule shells to obtain capsules.

Embodiment 11

The nanoparticles obtained in each of the embodiments 1 to 8 were added into a water solution for uniformly stirring to obtain a suspension. The suspension could be directly orally administered and could also be prepared into injections according to the preparation standard of the injections.

Embodiment 12

The nanoparticles obtained in each of the embodiments 1 to 8 and an appropriate amount of Witepsol were used for prepare a suppository.

**Claims**

1. Application of silicon dioxide aerogel as a nano-drug carrying system in pharmacy, wherein the nano-drug carrying system refers to a nanoparticle drug carrying system with the diameter of less than 100nm, which is formed in the form of adsorbing a carried drug in the holes of the silicon dioxide aerogel, and the silicon dioxide aerogel has the porosity of 95-99%, the aperture of 10-50nm, the specific surface area of 200-1000m$^2$/g, the density of 3-300kg/m$^3$ and the diameter of colloidal particles constituting a network of 1-50nm.

2. The application according to claim 1, **characterized in that** the application is the application in the preparation of oral preparations.

3. The application according to claim 2, **characterized in that** the application is the application in the preparation of anti-tumor drugs.

4. The application according to claim 1, **characterized in that** the mass ratio of the carried drug to the silicon dioxide aerogel is 1:0.5-20.

5. The application according to claim 1, **characterized in that** the silicon dioxide aerogel is hydrophilic silicon dioxide aerogel or the silicon dioxide aerogel has hydrophilic properties, which are obtained after heat treatment of a hydrophobic silicon dioxide aerogel.

6. The application according to claim 5, **characterized in that** the temperature for heat treatment is 300-1000°C.

7. The application according to claim 5, **characterized in that** when the carried drug is a soluble drug, the carried drug is firstly prepared into a saturated or unsaturated solution, and then the silicon dioxide aerogel is added for adsorption.

8. The application according to claim 5, **characterized in that** when the carried drug is a hardly soluble or insoluble drug, the carried drug is firstly prepared into a suspension, and then the silicon dioxide aerogel is added for adsorption or the carried drug and the silicon dioxide aerogel are prepared into the suspension together.

S4800 3.0kV 3.9mm x25.0k SE(U,LA0) 2012-11-22          2.00um

Fig.1

S4800 3.0kV 3.1mm x20.0k SE(M,LA0) 3/7/2012          2.00um

Fig.2

S4800 3.0kV 7.1mm x30.0k SE(U,LA0) 5/31/2011    1.00um

Fig.3

S4800 3.0kV 3.1mm x100k SE(M,LA0) 3/7/2012    500nm

Fig.4

Fig.5

Fig.6

EP 2 932 978 A1

Fig.7

Fig.8

24

Fig.9

Fig.10

EP 2 932 978 A1

Fig.11

Fig.12

Fig.13

Fig.14

<table>
<tr><td colspan="2" style="text-align:center"><strong>INTERNATIONAL SEARCH REPORT</strong></td><td>International application No.<br>PCT/CN2013/089312</td></tr>
</table>

**A. CLASSIFICATION OF SUBJECT MATTER**

See the extra sheet
According to International Patent Classification (IPC) or to both national classification and IPC

**B. FIELDS SEARCHED**

Minimum documentation searched (classification system followed by classification symbols)

IPC: A61K; A61P; C01B

Documentation searched other than minimum documentation to the extent that such documents are included in the fields searched

Electronic data base consulted during the international search (name of data base and, where practicable, search terms used)

DATABASE: CNABS, CPRSABS, DWPI, SIPOABS, CPEA, NCBI, MEDLINE, EMBASE, CA

SEARCH TERMS: silica aerogel, nano+, carrier, porosity, specific surface area, size, density, aperture, heat treatment, adsorption, hydrophilic+

**C. DOCUMENTS CONSIDERED TO BE RELEVANT**

| Category* | Citation of document, with indication, where appropriate, of the relevant passages | Relevant to claim No. |
|---|---|---|
| Y | ZENG, Miao et al., Adsorption and release of gentamicin sulfate for silica aerogels. JOURNAL OF THE CHINESE CERAMIC SORCIETY. August 2007, vol. 35, no. 8, pages 1081-1085, ISSN:0454-5648, see page 1082, the left column, paragraph [0006], page 1083, the right column, paragraphs [0002] and [0003] | 1-8 |
| Y | I. Smirnova et al. Adsorption of Drug on Silica Aerogels. Langmuire. 30 August 2003, ol. 19, no. 20, pages 8521-8525, ISSN: 0743-7463, see the abstract, page 8521, the left column, paragraph [0001], page 8521, the right column, paragraph [0001] to page 8522, right column, paragraph [0001] | 1-8 |

☒ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 05 March 2014 (05.03.2014) | 20 March 2014 (20.03.2014) |

| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>WEI, Yi<br><br>Telephone No. (86-10) 62412193 |

Form PCT/ISA /210 (second sheet) (July 2009)

| | INTERNATIONAL SEARCH REPORT | International application No. |
|---|---|---|
| | | PCT/CN2013/089312 |

C (Continuation).     DOCUMENTS CONSIDERED TO BE RELEVANT

| Category* | Citation of document, with indication, where appropriate, of the relevant passages | Relevant to claim No. |
|---|---|---|
| Y | I. Smirnova et al. Feasibility study of hydrophilic and hydrophobic silica aerogels as drug delivery systems. Journal of Non-Crystalline Solids. 15 December 2004, No. 350, pages 54-60, ISSN: 0022-3093, see the abstract, page 55, the right column, paragraph [0001] to page56, the right column, paragraph [0001] | 1-8 |
| Y | CHEN, Longwu et al. Aerogels. Chemistry. August 1997, pages 21-27, ISSN: 0441-3776, see page 23, paragraph [0002] | 1-8 |
| PX | CN 102961750 A (Graduate School at Shenzhen, Tsinghua University) 13 March 2013 (13.03.2013) , see claims 1-10, description, paragraphs [0012]-[0022], examples 1-10 | 1-8 |
| PX | CN 102961336 A (Graduate School at Shenzhen, Tsinghua University) 13 March 2013 (13.03.2013) , see claims 2, 3, 6 and 7, description, paragraph [0023], examples 1-8 | 1-8 |
| PX | CN 102961340 A (Graduate School at Shenzhen, Tsinghua University) 13 March 2013 (13.03.2013) , see claims 1, 2, 6, 7, 9 and 10, description, paragraph [0027], examples 1-9 | 1-8 |
| PX | CN 102961341 A (Graduate School at Shenzhen, Tsinghua University) 13 March 2013 (13.03.2013) , see claims 1-4, 6 and 7, description, paragraph [0022], examples 1-8 | 1-8 |
| PX | CN 102961342 A (Graduate School at Shenzhen, Tsinghua University) 13 March 2013 (13.03.2013) , see claims 1-6, description, paragraph [0021], examples 1-8 | 1-8 |
| PX | CN 102961343 A (Graduate School at Shenzhen, Tsinghua University) 13 March 2013 (13.03.2013) , see claims 1-4, 6 and 7, description, paragraph [0022], examples 1-8 | 1-8 |
| PX | CN 102961390 A (Graduate School at Shenzhen, Tsinghua University) 20 March 2013 (20.03.2013) , see claims 1-4, 6 and 7, description, paragraph [0026], examples 1-8 | 1-8 |
| PX | CN 102961391 A (Graduate School at Shenzhen, Tsinghua University) 20 March 2013 (20.03.2013) , see claims 1-4, 6 and 7, description, paragraph [0024], examples 1-8 | 1-8 |
| A | CN 101638237 A (ZHANG, Yi) 03 February 2010 (03.02.2010) , see the full text | 1-8 |
| A | CN 1865136 A (UNIV TONGJI) 22 November 2006 (22.11.2006) , see the whole text | 1-8 |

Form PCT/ISA /210 (continuation of second sheet) (July 2009)

## INTERNATIONAL SEARCH REPORT
### Information on patent family members

| | International application No. |
|---|---|
| | PCT/CN2013/089312 |

| Patent Documents referred in the Report | Publication Date | Patent Family | Publication Date |
|---|---|---|---|
| CN 102961750 A | 13.03.2013 | None | |
| CN 102961336 A | 13.03.2013 | None | |
| CN 102961340 A | 13.03.2013 | None | |
| CN 102961341 A | 13.03.2013 | None | |
| CN 102961342 A | 13.03.2013 | None | |
| CN 102961343 A | 13.03.2013 | None | |
| CN 102973490 A | 20.03.2013 | None | |
| CN 102973491 A | 20.03.2013 | None | |
| CN 101638237 A | 03.02.2010 | None | |
| CN 1865136 A | 22.11.2006 | None | |

Form PCT/ISA/210 (patent family annex) (July 2009)

## INTERNATIONAL SEARCH REPORT

International application No.

PCT/CN2013/089312

Continuation of classification of subject matter:

A61K 47/04 (2006.01) i

A61K 9/00 (2006.01) i

C01B 33/14 (2006.01) i

A61P 35/00 (2006.01) i

Form PCT/ISA /210 (extra sheet) (July 2009)